# EUROPEAN PATENT SPECIFICATION

(11) **EP 4 489 805 B1**
(45) Date of publication and mention of the grant of the patent: **15.07.2026**
(21) Application number: 23712063.9
(22) Date of filing: 08.03.2023
(51) Int. Cl.: A61L 29/04, A61L 29/08, A61L 29/14

(54) **INTERMITTENT CATHETERS**
INTERMITTIERENDE KATHETER
CATHÉTERS INTERMITTENTS

(30) Priority: 09.03.2022 US 202263318010 P
(43) Date of publication of application: 15.01.2025
(60) Divisional application: 26183646.4
(73) Proprietor: ConvaTec Limited, Deeside Industrial Park Deeside Flintshire CH5 2NU (GB)
(72) Inventor: PYTEL, Rachel, Deeside Flintshire CH5 2NU (GB); MOSSMAN, Paul, Deeside Flintshire CH5 2NU (GB); BARBIERI, Luca, Deeside Flintshire CH5 2NU (GB)
(74) Representative: Wilson Gunn
(86) International application number: PCT/GB2023/050546
(87) International publication number: WO 2023/170412

(56) References cited:
- US-A- 4 265 928
- US-A1- 2016 022 877
- US-A1- 2020 061 243

## Description

### Technical Field of the Invention

The present invention relates to negatively charged intermittent catheters, especially those presenting a surface negative charge after wetting with an aqueous medium.

### Background to the Invention

Intermittent urinary catheterisation is a process involving insertion of a urinary catheter through an individual's urethra and into their bladder, where it is retained to empty the bladder of urine for only the time period that is required for emptying, after which the catheter is removed. The process differs from long-term catheterisation, which makes use of an indwelling or Foley catheter that is inserted into the bladder for long periods of time (several days to months) to discharge the residual urine of the bladder continuously throughout the day.

Intermittent catheterisation is often used by patients suffering from abnormalities of the urinary system, resulting in urinary incontinence and/or a lack of control in permitting voluntary urination. Such individuals would typically make use of intermittent catheters several times a day.

Intermittent catheters are useful devices, providing users with independence and freedom to self-catheterise as and when required, without having to rely on trained personnel to be present. This, however, increases the need for intermittent catheters to be user friendly: in particular, both easy to insert and remove with minimum discomfort caused, and safe to use with features for minimising risk of infection. Users often report experiencing pain and discomfort upon insertion and/or removal of intermittent catheters. Users have, for instance, reported experiencing bladder spasms, burning sensations, and bleeding.

It is also easy for intermittent catheters to become contaminated and for bacteria to be introduced into the urethra and along the urinary tract. As a result, urinary tract infections (UTI) are common in individuals who practice intermittent catheterisation.

Surface coatings and additives for catheters have been used to help in alleviating some of these issues. US patents US 10 058 638 B2 and US 9 186 438 B2 describe the use of a catheter containing a polymer mixture of a base material and an amphiphilic block copolymer lubricious additive. The amphiphilic block copolymer contains both a hydrophobic and hydrophilic portion. The hydrophilic portion diffuses to the surface of the catheter due to incompatibility with the hydrophobic base material and provides for a lubricious surface coating. However, these patents do not properly address the issue of intermittent catheter bacterial contamination. US 2020/061243 A1 discloses an entirely or partially implantable medical product with a negatively charged surface for repulsing bacteria.

There exists a need for intermittent catheters that are both even easier and less painful to insert and remove; and that are safer to use, with features for minimising the risk of urethral microtrauma, bacterial contamination and infection, especially for non-medically trained individuals practising self-catheterisation.

It is an aim of embodiments of the present invention to address one or more of the above problems by providing an intermittent catheter, suitable for self-catheterisation use, which provides one or more of the following advantages:
- A high lubricity, non-stick surface making the intermittent catheter easier to insert and remove with a lower occurrence of microtraumas in patients.
- An antibiofouling surface which reduces the risk of the catheter being contaminated with bacteria when accidentally contacted by non-sterile objects, and which allows for reduced amounts of bacteria to be introduced into the urethra, thus reducing the risk of infections developing (particularly UTIs).
- Not overly complex design and simple/cost-effective to manufacture.

It is also an aim of embodiments of the invention to overcome or mitigate at least one problem of the prior art, whether expressly described herein or not.

### Summary of the Invention

According to a first aspect of the invention, there is provided an intermittent catheter comprising a hollow polymeric tubular body comprising a polymer that is negatively charged or that becomes negatively charged when wetted with an aqueous medium having a pH of greater than 2, said polymer being a derivative of at least one polyolefin material and/or at least one thermoplastic elastomeric material, and wherein the intermittent catheter further comprises at least one amphiphilic additive.

Such an intermittent catheter may provide at least one of the following advantages:
- The outer layer of the urothelium contains a glycosaminoglycan (GAG) layer that is believed to be negatively charged. The negative charge on the catheter polymer causes a repulsive interaction between the catheter body and the negatively charged GAG layer of the urothelium when the catheter is inserted, in use. This results in a low detachment force between the catheter and GAG layer, allowing the catheter to be inserted and removed smoothly and with minimal or no "sticking" to the urothelium, thus leading to minimal occurrence of urethral microtraumas.
- Where the catheter is wetted prior to use with an aqueous medium (e.g. a wetting agent), the negative charge on the catheter polymer is able to form charged hydrogen bonds with water molecules in the aqueous medium, which require higher detachment energy than neutral ones. This significantly reduces the speed at which the catheter dries, which keeps the catheter lubricated for longer and further decreases "sticking" effects between the catheter and the urothelium.
- The negative charge on the catheter polymer is also able to repel bacterial membranes which are typically negatively charged due to the presence of highly electronegative groups on their constituent phospholipids and lipopolysaccharides. The negatively charged catheter polymer helps minimise bacterial attachment to the catheter, greatly decreasing the risk of the user developing UTIs.
- The catheter of the invention may provide one or more of the above advantages through a greatly simplified structure relying only on the intrinsic nature of the catheter polymer. There is no reliance on the incorporation of additional additives and substances.

Overall, the polyolefin/thermoplastic elastomer derivative provides for a material with excellent lubricity and bacterial repulsion properties, and which further provides for an optimum balance of material rigidity and flexibility, making it especially well-suited for intermittent catheterisation applications.

In some embodiments, the polymer is or comprises a derivative of at least one polyolefin material, preferably comprising polyethylene and/or polypropylene. The polymer may be formed solely from a derivative of at least one polyolefin material.

In some embodiments, the polymer is or comprises a derivative of at least one thermoplastic elastomeric material. The polymer may be formed solely from a derivative of at least one thermoplastic elastomeric material.

In some embodiments, the polymer is or comprises a derivative of at least one thermoplastic elastomer of at least one material that is independently selected from the group consisting of: polyvinyl chloride, polytetrafluoroethylene, polyolefins, latex, silicones, synthetic rubbers, polyurethanes, polyesters, polyacrylates, polyamides, thermoplastic elastomeric materials, styrene block copolymers, polyether block amide, thermoplastic vulcanizates, thermoplastic copolyesters, thermoplastic polyamides, styrene-butadiene copolymer (SBC), styrene-ethylene-butylene-styrene copolymer (SEBS), water disintegrable or enzymatically hydrolysable material, and combinations, blends or copolymers of any of the above materials.

In preferred embodiments, the polymer is or comprises a derivative of at least one thermoplastic elastomer of at least one material that is independently selected from the group consisting of: polyolefins, polyesters, polyacrylates, polyamides, thermoplastic elastomeric material, polyether block amide, thermoplastic vulcanizates, thermoplastic copolyesters, thermoplastic polyamides, fluororubber, water disintegrable or enzymatically hydrolysable material, and combinations, blends or copolymers of any of the above materials.

In some embodiments, the polymer is or comprises a derivative of at least one thermoplastic elastomer of at least one water disintegrable or enzymatically hydrolysable material that is independently selected from the group consisting of: polyvinyl alcohol, extrudable polyvinyl alcohol, polyacrylic acids, polylactic acid, polyesters, polyglycolide, polyglycolic acid, poly lactic-co-glycolic acid, polylactide, amines, polyacrylamides, poly(*N*-(2-Hydroxypropyl) methacrylamide), starch, modified starches or derivatives, amylopectin, pectin, xanthan, scleroglucan, dextrin, chitosans, chitins, agar, alginate, carrageenans, laminarin, saccharides, polysaccharides, sucrose, polyethylene oxide, polypropylene oxide, acrylics, polyacrylic acid blends, poly(methacrylic acid), polystyrene sulfonate, polyethylene sulfonate, lignin sulfonate, polymethacrylamides, copolymers of aminoalkyl-acrylamides and methacrylamides, melamine-formaldehyde copolymers, vinyl alcohol copolymers, cellulose ethers, poly-ethers, polyethylene oxide, blends of polyethylene- polypropylene glycol, carboxymethyl cellulose, guar gum, locust bean gum, hydroxypropyl cellulose, vinylpyrrolidone polymers and copolymers, polyvinyl pyrrolidone-ethylene-vinyl acetate, polyvinyl pyrrolidone-carboxymethyl cellulose, carboxymethyl cellulose shellac, copolymers of vinylpyrrolidone with vinyl acetate, hydroxyethyl cellulose, gelatin, poly-caprolactone, poly(p-dioxanone), and combinations, blends or co-polymers of any of the above materials.

The polymer may preferably be or comprise a derivative of a thermoplastic polyolefin. The thermoplastic polyolefin may be independently selected from: polyethylene, polypropylene, or a combination thereof.

In some embodiments, the catheter polymer is formed of both a derivative of at least one polyolefin material and a derivative of at least one thermoplastic elastomeric material. The polymer derivatives may form part of a mixture. The thermoplastic elastomeric material may preferably be as described above. In some embodiments, at least 5 wt% of the catheter polymer is a derivative of a polyolefin material, or at least 10, 20, 30, 40, 50, 60, 70, 80, 90, or at least 95% of the catheter polymer is a derivative of a polyolefin material. In some embodiments, substantially 100% of the catheter polymer is a derivative of a polyolefin material.

In some embodiments, the polymer is negatively charged or becomes negatively charged when wetted with the aqueous medium, at and/or on a surface of the body. The surface may be an outer or an inner surface, preferably an outer surface.

The outer surface may comprise at least one of the group consisting of: the external facing surface of the body, the lumen of the body, and any eyelets present on the body. In preferred embodiments, the outer surface is the external-facing surface of the body and/or the inner lumen. In some embodiments, the outer surface may comprise the external-facing surface of the body of the catheter, the inner lumen, and the eyelets.

In some embodiments, the polymer is negatively charged or becomes negatively charged when wetted with the aqueous medium, at and/or on at least 25% of the outer surface area of the body, or at least 30, 35, 40, 45, 50, 55, 60, 65, 70, 75, 80, 85, 90, 95, 96, 97, 98, or at and/or on at least 99%, or substantially 100% of the outer surface area of the body. The polymer may be negatively charged or become negatively charged at and/or on no greater than 95%, or no greater than 90, 80, 70, 60, 50, or no greater than 40% of the outer surface area of the body.

The negative charge may be concentrated at and/or on the outer surface of the body. For example, at least 25%, or at least 30, 35, 40, 45, 50, 55, 60, 65, 70, 75, 80, 85, 90, 95, 96, 97, 98, or at least 99%, or substantially 100% of the negative charge may be at and/or on the outer surface of the body.

In some embodiments, the intermittent catheter polymer comprises at least one moiety that is negatively charged or becomes negatively charged when wetted with the aqueous medium.

In some embodiments, at least one moiety may be inherently negatively charged. The moiety may comprise a stabilised anion. The anion may be stabilised by one or more of the group consisting of: hyperconjugative/inductive effects, resonance/aromaticity effects, steric bulk, electronegative atoms, and combinations thereof.

In other embodiments, at least one moiety may become negatively charged when wetted with an aqueous medium having a pH of greater than 2, 2.5, or preferably of greater than 3, 3.5, 4, 4.5, 5, 5.5, 6, 6.5, 7, 7.5, 8, 8.5 or of greater than 9. In some embodiments, the aqueous medium may have a pH of no greater than 13, or of no greater than 12.5, 12, 11.5, 11, 10.5, or of no greater than 10. The aqueous medium may have a pH of between 2-13, 2-12, 2-11, 2-10, 2-9, 2-8, 2-7, or of between 3-13, 3-12, 3-11, 3-10, 3-9, 3-8, 3-7, or of between 4-13, 4-12, 4-11, 4-10, 4-9, 4-8, 4-7, or of between 5-13, 5-12, 5-11, 5-10, 5-9, 5-8, 5-7, or of between 6-13, 6-12, 6-11, 6-10, 6-9, 6-8, or of between 6-7. In such embodiments, at least one moiety may comprise an ionisable moiety that becomes negatively charged when wetted with the aqueous medium. The ionisable moiety may be a deprotonatable moiety (such as an acid group) that deprotonates and becomes negatively charged when wetted with the aqueous medium. In some embodiments, at least one moiety has a pKa of at least -10, or at least -8, -6, -4, -2, 0, 2, or at least 4. At least one moiety may have a pKa of no greater than 12, or preferably of no greater than 10, or of no greater than 8, or more preferably of no greater than 7, 6, 5, 4, 3, 2, or of no greater than 1. At least one moiety may have a pKa of between -10 and 12, or between -5 and 10, or between 0 and 8, or 0 and 7, or preferably between 1 and 7, or 1 and 6, or 2 and 7, or 2 and 6, or 1 and 5, or 2 and 5, or between 3 and 5.

The moiety may preferably comprise at least one electronegative atom, which may be independently selected from the group consisting of: oxygen, sulfur, nitrogen, a halogen, and combinations thereof. In some embodiments, the moiety is independently selected from the group consisting of: a carboxylic acid, a carboxylate, a sulfonic acid, a sulfonate, a phosphonic acid, a phosphonate, a stabilised phenolic acid, a stabilised phenolate, and derivatives thereof. Preferably, the moiety is a carboxylic acid or a carboxylate-containing moiety.

In some embodiments, the moiety is present in the intermittent catheter polymer at a total concentration of at least 0.02 wt%, or at least 0.04, 0.06, 0.08, 0.1, 0.5, 1, 2, 3, 4, 5, 6, 7, 8, 9, 10, 15, 20, 25, 30, 35, 40, 45, 50, 55, 60, 65, 70, 75, 80, 85, 90, or at least 95 wt%. In some embodiments, the moiety is present in the intermittent catheter polymer at a total concentration of no greater than 95 wt%, or of no greater than 90, 85, 80, 75, 70, 65, 60, 55, 50, 45, 40, 35, 30, 25, 20, 15, 10, or of no greater than 5 wt%.

In some embodiments, at least one moiety is embedded within the catheter body. In some embodiments, at least one moiety is present at and/or on a surface of the body, preferably the outer surface. In some embodiments, the catheter polymer has a polymer brush outer surface containing at least one moiety.

In some embodiments, at least 25%, or at least 30, 35, 40, 45, 50, 55, 60, 65, 70, 75, 80, 85, 90, 95, 96, 97, 98, or at least 99%, or substantially 100% of the moieties are at and/or on the outer surface of the body.

In some embodiments, the moieties are present at and/or on at least 5% of the outer surface area of the body, or at least 10, 15, 20, 25, 30, 35, 40, 45, 50, 55, 60, 65, 70, 75, 80, 85, 90, 95, 96, 97, 98, or at least 99%, or substantially 100% of the outer surface area of the body.

The aqueous medium may preferably have a pH as described above. The aqueous medium may be a liquid. The aqueous medium may be an aqueous solution or water and may optionally comprise one or more ingredients independently selected from the group consisting of: a salt, a buffer, an antibiotic, an active agent (which may be a medicament), a thickening agent, volatile agent, an alcohol, and combinations thereof. The aqueous medium may be a catheter wetting agent.

In some embodiments, the aqueous medium is a gel. The aqueous medium may be a colloid comprising particles dispersed in a liquid medium.

In some embodiments, the aqueous medium may have a viscosity of greater than 0.5 cP, or of greater than 1, 1.5, 2, 3, 4, 5, 10, 20, 30, 40, 50, 75, 100, 150, 200, 300, 400, 500, 600, 700, 800, 900, or of greater than 1000 cP. The aqueous medium may have a viscosity of no greater than 100000 cP, or of no greater than 90000, 80000, 70000, 60000, 50000, 40000, 30000, 20000, 10000, 5000, 4000, 3000, 2000, 1000, 500, 400, 300, 200, 100, 50, 25, 10, 5, 4, 3, 2, or of no greater than 1 cP. The aqueous medium may have a viscosity of between 0.5-5000 cP, or of between 0.5-2500, 0.5-1000, 1-1000, 10-1000, 50-1000, 100-1000, 500-1000, 0.5-500, 1-500, 10-500, 50-500, 100-500, 250-500, 0.5-250, 1-250, 10-250, 50-250, or of between 100-250 cP.

The aqueous medium may be applied, in use, to a surface of the intermittent catheter, preferably the outer surface. In some embodiments, the intermittent catheter is submerged in the aqueous medium.

At least one additive may be an A-B block copolymer comprising a hydrophobic hydrocarbon A-block and a hydrophilic B-block.

The hydrophobic A-block may comprise a carbon chain of at least 5 carbon atoms, or at least 10, 15, 20, 25, 30, 35, or 40 carbon atoms. The hydrophobic portion may preferably comprise a carbon chain of between 20-52 carbon atoms.

In some embodiments, the A-block comprises a hydrocarbon chain block of the formula CH₃CH₂(CH₂CH₂)ₐ. The value of "a" may be between 5-25; for instance, "a" may be 5, 6, 7, 8, 9, 10, 11, 12, 13, 14, 15, 16, 17, 18, 19, 20, 21, 22, 23, 24, or 25, or a half integer of any of the above values. The value of "a" may preferably be between 9-25; for instance, "a" may be 9, 10, 11, 12, 13, 14, 15, 16, 17, 18, 19, 20, 21, 22, 23, 24, or 25, or a half integer of any of the above values.

In some embodiments, the B-block is a hydrophilic oligomer comprising at least 1, 2, 3, 4, or at least 5 monomer units. In some embodiments, the B-block comprises no greater than 20, 19, 18, 17, 16, 15, 14, 13, 12, 11, 10, 9, 8, 7, or no greater than 6 monomer units. In some embodiments, the B-block comprises between 2 and 15 monomer units, preferably between 2 and 10 monomer units.

The B-block monomer units may be derived from at least one monomer independently selected from the group consisting of: alkylene oxides, alkylene glycols, epihalohydrins, unsaturated carboxylic acids, alkylene imines, lactones, vinyl alcohol, and vinyl alkanoates. The monomer may be preferably selected from the group consisting of: ethylene oxide, propylene oxide, ethylene glycol, propylene glycol, epichlorohydrin, acrylic acid, methacrylic acid, ethylene imine, caprolactone, vinyl alcohol, and vinyl acetate. In some embodiments, the additive comprises a poly (alkylene oxide) B-block formed from alkylene oxide monomer units that are independently selected from the group consisting of: ethylene oxide, propylene oxide, and combinations thereof. In preferred embodiments, all of the monomer units are ethylene oxide or all of the monomer units are propylene oxide.

In some embodiments, one or both of the hydrophobic hydrocarbon A-block and the hydrophilic B-block may be branched. The hydrophobic A-block may comprise hydrophobic hydrocarbon chains branching therefrom. The hydrophobic hydrocarbon chains may be of shorter chain lengths than the hydrophobic hydrocarbon A-block. The hydrophilic B-block may comprise further hydrophilic B-blocks branching therefrom.

In some embodiments, the additive is homogenously distributed with the polymer. The additive may be uniformly distributed throughout the polymer.

At least some of the additive may be at or on the outer surface of the body. By "at the outer surface", it is meant that at least a portion of the additive forms part of the surface or protrudes from the surface. In some embodiments, part of the additive is retained or anchored in the body while part of the additive forms part of or protrudes from the outer surface of the body.

The additive may be concentrated at or on the outer surface of the body. For example, at least 25%, 30%, 35%, 40%, 45%, 50%, 55%, 60%, 65%, 70%, 75%, 80%, 85%, 90%, or at least 95% of the number of molecules of the additive may be at or on the outer surface of the body.

In some embodiments, at least 25%, 30%, 35%, 40%, 45%, 50%, 55%, 60%, 65%, 70%, 75%, 80%, 85%, 90%, or at least 95% of the number of molecules of additive may have hydrophilic portions that are at or on the outer surface of the body.

In some embodiments, the additive is located at and/or on at least 50, 55, 60, 65, 70, 75, 80, 85, 90, 95, 96, 97, 98 or at least 99% of the outer surface area of the polymeric tubular body, preferably at least 75% or at least 90% of the outer surface area of the polymeric tubular body or between 75% and 100% of the outer surface area.

In some embodiments, the additive is present at a concentration of at least 0.1, 0.2, 0.3. 0.4. 0.5, 0.75, 1, 2, 3, 4, 5, 10, 15 or at least 20% by weight of the combination of catheter polymer and additive. The additive may be present a concentration of between 0.1-20%, and more preferably between 0.5-15% or 0.5-5% by weight of the combination of catheter polymer and additive.

In some embodiments, the additive comprises a layer that is on or that comprises a surface of the body, preferably the outer surface.

The layer comprising the additive may be on the surface of the body. In some embodiments, the layer comprising the additive is substantially separate from the body and the layer may be bonded to the body. The layer may be bonded to the body via covalent bonds, ionic bonds, hydrogen bonds, or Van der Waals forces. The additive may be bonded to the body via one or more surface linker groups which may be present on the additive, the body of the intermittent catheter or both.

In some embodiments, the layer comprising the additive may comprise the surface of the body. In such embodiments the layer may form the surface of the body. The layer may comprise a co-extruded layer which is melded with or is physically entangled with the body, and this may form an integral layer. The layer of additive may be integrally formed with the body.

In some embodiments, polymer diffusion occurs between the layer comprising the additive and the catheter body. The layer and the body may be held together by polymer chains extending across the interface between the layer and body. In some embodiments, the additive infiltrates the catheter body.

In some embodiments, the layer comprising the additive comprises or is on an inner surface of the body, an outer surface of the body, or both. The inner surface of the body may comprise a lumen of the intermittent catheter. In preferred embodiments, the layer comprising the additive comprises or is on at least an outer surface of the body.

In some embodiments, the layer comprising the additive is on or comprises at least 50, 55, 60, 65, 70, 75, 80, 85, 90, 95, 96, 97, 98 or at least 99% of the or each surface area of the body, preferably at least 75% or at least 90% of the or each surface area or between 75% and 100% of the or each surface area. In embodiments in which the layer comprising the additive comprises or is on both an inner and outer surface of the body, the additive may comprise at least 50, 55, 60, 65, 70, 75, 80, 85, 90, 95, 96, 97, 98 or at least 99% of each surface area of the body, preferably at least 75% or at least 90% of each surface area or between 75% and 100% of each surface area of both surfaces.

In some embodiments, at least 75% of the layer comprising the additive, or at least 76, 77, 78, 79, 80, 81, 82, 83, 84, 85, 86, 87, 88, 89, 90, 91, 92, 93, 94, 95, 96, 97, 98, 99, or 100% of the layer is the additive.

In some embodiments, the layer comprising the additive has an additive concentration of at least 0.1, 0.2, 0.3. 0.4. 0.5, 0.75, 1, 2, 3, 4, 5, 10, 15 or at least 20% by weight of the combination of catheter polymer and additive.

In some embodiments, the layer comprising the additive has an additive concentration of no greater than 70, 65, 60, 65, 60, 55, or of no greater than 50% by weight of the combination of the catheter polymer and additive.

The layer comprising the additive may have an additive concentration of greater than 5% by weight of the combination of the catheter polymer and additive. The layer may have an additive concentration of between 6-50% by weight of the combination of the catheter polymer and additive.

The layer comprising the additive may have an additive concentration of between 10-50% by weight of the combination of the catheter polymer and additive, or of between 15-50, 20-50, 25-50, 30-50, 35-50, 40-50, or of between 45-50% by weight of the combination of the catheter polymer and additive.

The layer comprising the additive may have an additive concentration of between 6-45% by weight of the combination of the catheter polymer and additive, or of between 6-40, 6-35, 6-30, 6-25, 6-20, 6-15, or of between 6-10% by weight of the combination of the catheter polymer and additive.

The layer comprising the additive may have an additive concentration of between 10-45% by weight of the combination of the catheter polymer and additive, or of between 15-45, 20-45, 25-45, 30-45, 35-45, 40-45, 10-40, 15-40, 20-40, 25-40, 30-40, 35-40, 10-35, 15-35, 20-35, 25-35, 30-35, 10-30, 15-30, 20-30, 25-30, 10-25, 15-25, 20-25, 10-20, 15-20, or of between 10-15% by weight of the combination of the catheter polymer and additive.

In some embodiments, the layer comprising the additive has a thickness of at least 1 µm, or of at least 2, 3, 4, 5, 6, 7, 8, 9, 10, 15, 20, 25, 30, 35, 40, 45, or of at least 50 µm.

In some embodiments, the layer comprising the additive has a thickness of no more than 10000 µm, or of no more than 9000, 8000, 7000, 6000, 5000, 4000, 3000, 2000, 1000, 900, 800, 700, 600, 500, 400, or of no more than 300 µm.

In some embodiments, the layer comprising the additive has a thickness of between 50-300 µm.

The layer comprising the additive may have a thickness of between 60-300 µm, or of between 80-300, 100-300, 120-300, 140-300, 160-300, 180-300, 200-300, 220-300, 240-300, 260-300, or of between 280-300 µm.

The layer comprising the additive may have a thickness of between 50-280 µm, or of between 50-260, 50-240, 50-220, 50-200, 50-180, 50-160, 50-140, 50-120, 50-100, 50-80, or of between 50-60 µm.

The layer comprising the additive may have a thickness of between 60-280 µm, or of between 80-280, 100-280, 120-280, 140-280, 160-280, 180-280, 200-280, 220-280, 240-280, 260-280, 60-260, 80-260, 100-260, 120-260, 140-260, 160-260, 180-260, 200-260, 220-260, 240-260, 60-240, 80-240, 100-240, 120-240, 140-240, 160-240, 180-240, 200-240, 220-240, 60-220, 80-220, 100-220, 120-220, 140-220, 160-220, 180-220, 200-220, 60-200, 80-200, 100-200, 120-200, 140-200, 160-200, 180-200, 60-180, 80-180, 100-180, 120-180, 140-180, 160-180, 60-160, 80-160, 100-160, 120-160, 140-160, 60-140, 80-140, 100-140, 120-140, 60-120, 80-120, 100-120, 60-100, 80-100, or of between 60-80 µm.

In some embodiments, the polymeric tubular body comprises a separate or further lubricating agent or bacteria-repellent agent at and/or on a surface of the body in addition to the additive, preferably at and/or on the outer surface. The separate or further lubricating agent or bacteria-repellent agent may be bonded at and/or on the surface.

In some embodiments, said further lubricating agent or bacteria-repellent agent is formed from a coating material selected from the group consisting of: silver-based, polytetrafluoroethylene, hydrogel, silicone, lecithin, salicylic acid, minocycline, rifampin, fluorinated ethylene propylene, polyvinylidone, polyvinyl compounds, polylactames, polyvinyl pyrrolidones, polysaccharides, heparin, dextran, xanthan gum, derivatised polysaccharides, hydroxy propyl cellulose, methyl cellulose, polyurethanes, polyacrylates, polyhydroxyacrylates, polymethacrylates, polyacrylamides, polyalkylene oxides, polyethylene oxides, polyvinyl alcohols, polyamides, polyacrylic acid, hydroxy ethylmethyl acrylate, polymethylvinyl ether, maleinic acid anyhydride, penicillin, neomycin sulfate, cephalothin, Bacitracin, phenoxymethyl penicillin, lincoymycin hydrochloride, sulfadiazine, methyl sulfadiazine, succinoylsulfathiazole, phthalylsulfathiazde, sulfacetamine, procaine penicillin, streptomycin, aureomycin, terramycin, terramycin, quaternary ammonium halides, cetyl pyridinium chloride, triethyl dodecyl ammonium bromide, hexachlorophene and nitrofurazone, or any combination thereof.

According to a second aspect of the invention, there is provided a method of manufacturing the intermittent catheter of the first aspect of the invention, the method comprising the step of: incorporating a moiety into an intermittent catheter polymer comprising at least one polyolefin material and/or at least one thermoplastic elastomeric material, wherein the moiety is negatively charged or becomes negatively charged when wetted with an aqueous medium having a pH of greater than 2.

Statements of invention above relating to the first aspect of the invention may also be applied to the second aspect of the invention.

In some embodiments, the method comprises treating the intermittent catheter polymer, and preferably an outer surface thereof to introduce at least one moiety that is negatively charged or that becomes negatively charged when wetted, or to prepare the catheter polymer for introduction of the moiety. The treatment may include one or more of the group consisting of: corona treatment, plasma treatment, flame treatment, and radiation treatment (including treatment of the polymer with high energy ionising radiation, such as gamma rays, X-rays, or high speed electrons). Such methods are particularly useful for polyolefin and thermoplastic elastomer catheter materials and surfaces. In some embodiments, the treatment is performed in the presence of a molecule that comprises a moiety that is negatively charged or that becomes negatively charged when wetted, or in the presence of a molecule that is configured to react with the treated catheter polymer to provide such a moiety. In a particular embodiment, the molecule is carbon dioxide, preferably in the form of carbon dioxide gas.

Such methods allow for modification of the intermittent catheter polymer to incorporate moieties that are negatively charged or that become negatively charged when wetted with the aqueous medium.

In some embodiments, the method comprises surface oxidation of the intermittent catheter polymer, preferably of an outer surface thereof. The method may comprise cleaving polymer chains at or on the surface of the intermittent catheter and incorporating moieties that are negatively charged or that become negatively charged when wetted with the aqueous medium.

In some embodiments, the method comprises incorporating the moieties that are negatively charged or that become negatively charged onto the intermittent catheter polymer surface via radical grafting. The method may comprise initiating radical grafting by treatment of the polymer surface with ultraviolet radiation. The method may comprise treating the polymer surface with ultraviolet radiation in the presence of a molecule, preferably an olefin, comprising a moiety that is negatively charged or that becomes negatively charged when contacted with the aqueous medium. In such embodiments, the catheter polymer material may comprise a polyolefin, such as polyethylene and/or polypropylene.

In some embodiments, the method comprises tethering at least one polymer comprising a moiety that is negatively charged or that becomes negatively charged when wetted, to the intermittent catheter polymer and preferably to an outer surface thereof. The method may comprise tethering multiple polymers comprising such moieties to the outer surface of the catheter polymer. The tethered polymers may form a polymer brush that is negatively charged or that becomes negatively charged when wetted with an aqueous medium.

In some embodiments, the method comprises incorporating the moiety into the intermittent catheter polymer before formation of the intermittent catheter into a tubular catheter body. In some embodiments, the method comprises incorporating the moiety into the intermittent catheter polymer after formation of the catheter into the tubular catheter body. The method preferably comprises incorporating at least one moiety at and/or on a surface of the catheter body, preferably the outer surface. Amounts of moieties at and/or on the surface are preferably as described for the first aspect of the invention above.

The method may comprise providing the intermittent catheter polymer and the additive, and forming a hollow polymeric tubular catheter body comprising the catheter polymer and the additive.

Formation of the hollow polymeric tubular body may comprise a melt-extrusion or injection-moulding procedure. In some embodiments, the method may comprise mixing the catheter polymer and additive together to form a mixture, and melt-extruding or injection-moulding the mixture to form the catheter body.

The catheter polymer and/or additive, preferably both, may be provided in granulate or powder form. The method may comprise mixing the granulate or powder catheter polymer and additive to form a mixture, and melt-extruding or injection-moulding the mixture to form the hollow polymeric tubular intermittent catheter body.

In some embodiments, the method comprises melting the mixture of the catheter polymer and additive to form a second mixture before melt-extruding or injection-moulding the second mixture to form the hollow polymeric tubular intermittent catheter body.

The method may comprise extruding the catheter polymer and the additive to form a hollow polymeric tubular body comprising the catheter polymer and a layer comprising the additive on or comprising a surface of the catheter body, preferably an outer surface.

In some embodiments, the method comprises co-extruding the catheter polymer and the layer comprising the additive simultaneously.

In some embodiments, the method comprises co-extruding the layer comprising the additive using direct, indirect, hydrostatic, or impact co-extrusion.

In some embodiments, the method comprises co-extruding the layer comprising the additive using cold, warm, hot, or friction co-extrusion.

In other embodiments, the method comprises extrusion coating the additive on the surface of the catheter body. The surface preferably comprises an outer surface of the body.

The catheter polymer and/or additive, preferably both, may be provided in granulate or powder form prior to extrusion.

The method may comprise melt-extruding the catheter polymer to form the hollow polymeric tubular body, and separately melt-extruding the additive onto a surface (preferably the outer surface) of the hollow polymeric tubular body.

The method may comprise using a blown or cast film process to coat the layer comprising an additive as a molten web of synthetic resin onto the surface of the intermittent catheter body after its formation.

The method may comprise extruding a molten layer comprising an additive from a slot die directly onto a tacky catheter body. The tacky catheter body may be moved beneath the die on extrusion coating of the additive to form the layer comprising an additive on the outer surface of the catheter.

Alternatively, the method may comprise co-extruding (melt extruding) both the catheter polymer and additive substantially simultaneously, so that a layer comprising the additive is formed as a layer on the catheter polymer, or comprises the surface of the catheter polymer.

The method may comprise melting both a catheter polymer mixture and additive and delivering a steady volumetric throughput of both the mixture and additive to a single extrusion head under pressure, which allows for co-extrusion of the layer comprising an additive and the catheter polymer simultaneously. The co-extrusion may employ elevated temperature and pressure causing entanglements to form between the catheter polymer chains and additive molecules.

The method may comprise mixing the granulate or powder catheter polymer with a further additive, as described in the second aspect of the invention, to form a mixture, and melt-extruding the mixture to form the hollow polymeric tubular body.

In some embodiments, the method comprises melting the mixture of the catheter polymer and further additive to form a second mixture before extruding the second mixture to form the hollow polymeric tubular intermittent catheter body.

According to a third aspect of the invention, there is provided the use of a polymer derivative of at least one polyolefin material and/or at least one thermoplastic elastomeric material that is negatively charged or that becomes negatively charged when wetted with an aqueous medium having a pH of greater than 2, as a bacteria-repellent and/or lubricant in or on the intermittent catheter of the first aspect of the invention.

The polymer derivative is preferably the catheter polymer of the first aspect of the invention. Statements of invention relating to the first aspect of the invention above may also be applied to the third aspect of the invention.

The intermittent catheter may be manufactured according to the method of the second aspect of the invention.

According to a fourth aspect of the invention, there is provided a packaged intermittent catheter comprising a packaging container in which is located an intermittent catheter of the first aspect of the invention, and optionally a wetting agent.

Statements of invention above relating to the intermittent catheter of the first aspect of the invention or to any of its components may also be applied to the fourth aspect of the invention.

The wetting agent, when present, may surround the intermittent catheter or may be separated from the intermittent catheter within the packaging, for example by providing the wetting agent in a separate container within the packaging container.

The wetting agent may comprise the aqueous medium of the first aspect of the invention. Statements of invention above relating to the aqueous medium may also be applied to the fourth aspect of the invention.

In some embodiments, the aqueous medium is contained within a separate container, such as a bag or sachet, within the container housing and is not in direct contact with the intermittent catheter.

The separate container may be pierceable, in use, to release the contained aqueous medium from the separate container and into direct contact with the intermittent catheter.

Prior to at least one of opening the packaging, removing the catheter from the packaging and inserting the intermittent catheter, the user may release the aqueous medium from the separate container and apply the medium to the outer surface of the catheter.

### Detailed Description of the Invention

In order that the invention may be more clearly understood embodiments thereof will now be described, by way of example only:

### Example 1:

A first embodiment of an intermittent catheter not of the invention is provided by an intermittent catheter containing a hollow polymeric tubular body comprising a polymer formed of thermoplastic polyethylene that is modified on its outer surface to include carboxylic acid moieties.

The intermittent catheter body may be prepared by extrusion or injection moulding, as described in US patents US 10 058 638 B2 and US 9 186 438 B2. After forming the catheter body, the carboxylic acid moieties were introduced on the outer surface of the body by a UV initiated graft method. The outer surface was treated with high energy UV radiation in the presence acrylic acid molecules. The UV irradiation activated the surface of the catheter, which was then able to react with the acrylic acid monomers to graft them to the surface. The resulting surface was modified with carboxylic acid moieties.

Before use, an aqueous medium with a pH of greater than 2 (preferably greater than 3.5), which may also function as a wetting agent, is contacted with the outer surface of the intermittent catheter. This may be performed by the user shortly before use or the intermittent catheter may alternatively be packaged submerged in the aqueous medium and ready for use.

The aqueous medium deprotonates the surface carboxylic acid moieties to generate negatively charged carboxylates.

The carboxylate moieties provide at least the following advantages:
- The negative charge on the carboxylate repels the negatively charged GAG layer of the urothelium when the catheter is inserted. This results in a low detachment force between the additive and GAG layer, which allows the catheter to be inserted and removed smoothly and with minimal "sticking" of the additive to the urothelium, thus decreasing the occurrence of urethral microtraumas.
- The negative charge is also able to form strong charged hydrogen bonds with water molecules in the aqueous medium. This reduces the speed at which the catheter dries and keeps the catheter lubricated for longer, further decreasing "sticking" effects between the catheter and the urothelium.
- The negative charge is also able to repel negatively charged bacterial membranes, which helps minimise bacterial attachment to the catheter, greatly decreasing the risk of the user developing UTIs.

The intermittent catheter of Example 1 was less susceptible to bacterial attachment compared with a similar unmodified catheter and a modified catheter of a non-polyolefin/non-thermoplastic elastomer material. The catheter of Example 1 was also much easier and less painful to insert and remove, in use.

### Example 2:

A first embodiment of an intermittent catheter of the invention is provided by an intermittent catheter containing a hollow polymeric tubular body comprising a polymer formed of polypropylene that is modified on its outer surface to include carboxylic acid moieties. The catheter body further comprises an amphiphilic additive of the formula CH₃CH₂(CH₂CH₂)₁₅(OCH₂CH₂)₅OH.

The intermittent catheter may be prepared as described in US patents US 10 058 638 B2 and US 9 186 438 B2. After forming the catheter body comprising the additive, the carboxylic acid moieties were introduced on the outer surface of the catheter polymer by a plasma treatment method. Plasma treatment of the outer surface of the catheter was performed in the presence of carbon dioxide gas, which resulted in incorporation of carboxylic acid moieties on the outer surface of the catheter polymer.

The catheter is contacted with an aqueous medium prior to use, as in Example 1 above, after which the catheter is used in the conventional manner.

Like Example 1, the intermittent catheter of Example 2 was less susceptible to bacterial attachment and was much easier and less painful to insert and remove, in use, compared with a similar unmodified catheter and a modified catheter of a non-polyolefin/non-thermoplastic elastomer material.

The polyethylene oxide hydrophilic portion of the amphiphilic additive further enhanced the lubricity of the catheter. The hydrophilic portion seeks towards the outer surface of the body due to its incompatibility with the hydrophobic catheter polymer.

The above embodiments are described by way of example only. Many variations are possible without departing from the scope of the invention as defined in the appended claims.

## Claims

1. An intermittent catheter comprising a hollow polymeric tubular body comprising a polymer that is negatively charged or that becomes negatively charged when wetted with an aqueous medium having a pH greater than 2, said polymer being a derivative of at least one polyolefin material and/or at least one thermoplastic elastomeric material, and wherein the intermittent catheter further comprises at least one amphiphilic additive.

2. An intermittent catheter as claimed in claim 1, wherein the polymer is or comprises a derivative of at least one thermoplastic elastomer of at least one material that is independently selected from the group consisting of: polyolefins, polyesters, polyacrylates, polyamides, polyether block amide, thermoplastic vulcanizates, thermoplastic copolyesters, thermoplastic polyamides, fluororubber, water disintegrable or enzymatically hydrolysable material, and combinations, blends or co-polymers of any of the above materials.

3. An intermittent catheter as claimed in any preceding claim, wherein the polymer is or comprises a derivative of at least one polyolefin material, preferably comprising polyethylene and/or polypropylene.

4. An intermittent catheter as claimed in any preceding claim, wherein the polymer is negatively charged or becomes negatively charged when wetted with an aqueous medium having a pH of greater than 2, at and/or on a surface, and preferably an outer surface of the body, more preferably at least 50% of the outer surface of the body.

5. An intermittent catheter as claimed in any preceding claim, wherein the intermittent catheter polymer comprises at least one moiety that is negatively charged or becomes negatively charged when wetted with an aqueous medium having a pH of greater than 2, wherein the at least one moiety is preferably present in the intermittent catheter polymer at a total concentration of at least 0.5 wt.%, and wherein at least one of the moieties is preferably present at and/or on a surface of the body, preferably the outer surface, and more preferably at least 50% of the outer surface area of the body, and wherein at least 50% of the moieties are preferably at and/or on the outer surface of the body.

6. An intermittent catheter as claimed in any preceding claim, wherein the polymer comprises at least one ionisable moiety, preferably at least one deprotonatable moiety, that becomes negatively charged when wetted with an aqueous medium having a pH of greater than 2.

7. An intermittent catheter as claimed in any preceding claim, wherein the at least one additive is an A-B block copolymer comprising a hydrophobic hydrocarbon A-block and a hydrophilic B-block.

8. An intermittent catheter as claimed in claim 7, wherein the A-block comprises a hydrocarbon chain block of the formula CH₃CH₂(CH₂CH₂)ₐ where "a" is 5-25, and preferably 9-25.

9. An intermittent catheter as claimed in claim 7 or 8, wherein the B-block is a hydrophilic oligomer comprising between 2 and 10 monomer units optionally derived from at least one monomer independently selected from the group consisting of: alkylene oxides, alkylene glycols, epihalohydrins, unsaturated carboxylic acids, alkylene imines, lactones, vinyl alcohol, and vinyl alkanoates.

10. An intermittent catheter as claimed in any preceding claim, wherein the at least one additive is homogenously distributed with the polymer.

11. An intermittent catheter as claimed in any preceding claim, wherein the at least one additive comprises a layer that is on a surface or comprises a surface of the body, preferably the outer surface.

12. An intermittent catheter as claimed in any preceding claim, wherein the at least one additive is at and/or on at least 50% of the outer surface area of the polymeric tubular body.

13. An intermittent catheter as claimed in any preceding claim, wherein the polymer that is negatively charged or that becomes negatively charged when wetted with an aqueous medium having a pH greater than 2 is the polymer of the catheter body, and wherein the polymer of the catheter body is optionally a derivative of at least one polyolefin material.

14. A method of manufacturing an intermittent catheter according to any one of claims 1 to 13 comprising the step of: incorporating a moiety into an intermittent catheter polymer comprising at least one polyolefin material and/or at least one thermoplastic elastomeric material, wherein the moiety is negatively charged or becomes negatively charged when wetted with an aqueous medium having a pH of greater than 2.

15. Use of a polymer derivative of at least one polyolefin material and/or at least one thermoplastic elastomeric material that is negatively charged or that becomes negatively charged when wetted with an aqueous medium having a pH of greater than 2, as a bacteria-repellent and/or lubricant in or on an intermittent catheter according to any one of claims 1 to 13.

## Patentansprüche

1. Intermittierender Katheter, aufweisend einen hohlen, polymeren, röhrenförmigen Körper, der ein Polymer aufweist, das negativ geladen ist oder sich negativ auflädt, wenn es mit einem wässrigen Medium mit einem pH-Wert größer als 2 benetzt wird, wobei das Polymer ein Derivat wenigstens eines Polyolefinmaterials und/oder wenigstens eines thermoplastischen elastomeren Materials ist, und wobei der intermittierende Katheter weiterhin wenigstens ein amphiphiles Additiv aufweist.

2. Intermittierender Katheter nach Anspruch 1, wobei das Polymer ein Derivat wenigstens eines thermoplastischen Elastomers aus wenigstens einem Material ist oder dieses aufweist, wobei das Material unabhängig ausgewählt ist aus der Gruppe bestehend aus: Polyolefinen, Polyestern, Polyacrylaten, Polyamiden, Polyetherblockamiden, thermoplastischen Vulkanisaten, thermoplastischen Copolyestern, thermoplastischen Polyamiden, Fluorkautschuk, wasserlöslichem oder enzymatisch hydrolysierbarem Material sowie Kombinationen, Mischungen oder Copolymeren beliebiger der vorgenannten Materialien.

3. Intermittierender Katheter nach einem der vorhergehenden Ansprüche, wobei das Polymer ein Derivat wenigstens eines Polyolefinmaterials ist oder dieses aufweist, vorzugsweise aufweisend Polyethylen und/oder Polypropylen.

4. Intermittierender Katheter nach einem der vorhergehenden Ansprüche, wobei das Polymer negativ geladen ist oder sich negativ auflädt, wenn es mit einem wässrigen Medium mit einem pH-Wert größer als 2 benetzt wird, an und/oder auf einer Oberfläche, vorzugsweise einer Außenoberfläche des Körpers, besonders bevorzugt auf mindestens 50 % der Außenoberfläche des Körpers.

5. Intermittierender Katheter nach einem der vorhergehenden Ansprüche, wobei das Polymer des intermittierenden Katheters wenigstens einen Anteil aufweist, der negativ geladen ist oder sich negativ auflädt, wenn dieser mit einem wässrigen Medium mit einem pH-Wert größer als 2 benetzt wird, wobei der wenigstens eine Anteil in dem Polymer des intermittierenden Katheters vorzugsweise in einer Gesamtkonzentration von mindestens 0,5 Gew.-% vorhanden ist, und wobei wenigstens einer der Anteile vorzugsweise an und/oder auf einer Oberfläche des Körpers vorliegt, vorzugsweise der Außenoberfläche, und besonders bevorzugt auf wenigstens 50 % der Außenoberfläche des Körpers, und wobei wenigstens 50 % der Anteile vorzugsweise an und/oder auf der Außenoberfläche des Körpers vorliegt.

6. Intermittierender Katheter nach einem der vorhergehenden Ansprüche, wobei das Polymer wenigstens einen ionisierbaren Anteil, vorzugsweise wenigstens einen deprotonierbaren Anteil, aufweist, der sich negativ auflädt, wenn dieser mit einem wässrigen Medium mit einem pH-Wert größer als 2 benetzt wird.

7. Intermittierender Katheter nach einem der vorhergehenden Ansprüche, wobei das wenigstens eine Additiv ein A-B-Blockcopolymer ist, das einen hydrophoben Kohlenwasserstoff-A-Block und einen hydrophilen B-Block aufweist.

8. Intermittierender Katheter nach Anspruch 7, wobei der A-Block einen Kohlenwasserstoffkettenblock der Formel CH₃CH₂ (CH₂CH₂)ₐ aufweist, wobei "a" 5-25, und vorzugsweise 9-25 ist.

9. Intermittierender Katheter nach Anspruch 7 oder 8, wobei der B-Block ein hydrophiles Oligomer ist, das zwischen 2 und 10 Monomereinheiten aufweist, die optional von wenigstens einem Monomer abgeleitet sind, das unabhängig ausgewählt ist aus der Gruppe bestehend aus: Alkylenoxiden, Alkylenglykolen, Epihalohydrinen, ungesättigten Carbonsäuren, Alkyleniminen, Lactonen, Vinylalkohol und Vinylalkanoaten.

10. Intermittierender Katheter nach einem der vorhergehenden Ansprüche, wobei das wenigstens eine Additiv mit dem Polymer gleichmäßig verteilt ist.

11. Intermittierender Katheter nach einem der vorhergehenden Ansprüche, wobei das wenigstens eine Additiv eine Schicht aufweist, die sich auf einer Oberfläche des Körpers befindet oder eine Oberfläche des Körpers aufweist, vorzugsweise die Außenoberfläche.

12. Intermittierender Katheter nach einem der vorhergehenden Ansprüche, wobei sich das wenigstens eine Additiv auf und/oder an wenigstens 50 % der Außenoberfläche des polymeren röhrenförmigen Körpers befindet.

13. Intermittierender Katheter nach einem der vorhergehenden Ansprüche, wobei das Polymer, das negativ geladen ist oder sich negativ auflädt, wenn es mit einem wässrigen Medium mit einem pH-Wert größer als 2 benetzt wird, das Polymer des Katheterkörpers ist, und wobei das Polymer des Katheterkörpers optional ein Derivat wenigstens eines Polyolefinmaterials ist.

14. Verfahren zur Herstellung eines intermittierenden Katheters nach einem der Ansprüche 1 bis 13, umfassend den Schritt: Einbringen eines Anteils in ein intermittierendes Katheterpolymer, aufweisend wenigstens ein Polyolefinmaterial und/oder wenigstens ein thermoplastisches elastomeres Material, wobei der Anteil negativ geladen ist oder negativ geladen wird, wenn dieser mit einem wässrigen Medium mit einem pH-Wert größer als 2 benetzt wird.

15. Verwendung eines Polymerderivats wenigstens eines Polyolefinmaterials und/oder wenigstens eines thermoplastischen elastomeren Materials, das negativ geladen ist oder sich negativ auflädt, wenn es mit einem wässrigen Medium mit einem pH-Wert größer als 2 benetzt wird, als bakterienabweisendes und/oder schmierendes Mittel in oder auf einem intermittierenden Katheter nach einem der Ansprüche 1 bis 13.

## Revendications

1. Un cathéter intermittent comprenant un corps tubulaire creux polymère comprenant un polymère, qui est chargé négativement ou qui devient chargé négativement lorsqu'il est humidifié par un fluide aqueux d'un pH supérieur à 2, ledit polymère étant un dérivé d'au moins une matière polyoléfinique et/ou d'au moins une matière élastomère thermoplastique, et dans lequel le cathéter intermittent comprend en outre au moins un additif amphiphile.

2. Un cathéter intermittent tel que revendiqué dans la revendication 1, dans lequel le polymère est ou comprend un dérivé d'au moins un élastomère thermoplastique d'au moins une matière, qui est choisie indépendamment dans le groupe consistant en : des polyoléfines, des polyesters, des polyacrylates, des polyamides, un polyéther séquencé amide, des vulcanisats thermoplastiques, des copolyesters thermoplastiques, des polyamides thermoplastiques, du fluorocaoutchouc, de la matière désintégrable ou hydrolysable enzymatiquement, et des combinaisons, mélanges ou copolymères de n'importe lesquelles des matières ci-dessus.

3. Un cathéter intermittent tel que revendiqué dans l'une quelconque des revendications précédentes, dans lequel le polymère et/ou comprend un dérivé d'au moins une matière polyoléfinique en comprenant, de préférence, du polyéthylène et/ou du polypropylène.

4. Un cathéter intermittent tel que revendiqué dans l'une quelconque des revendications précédentes, dans lequel le polymère est chargé négativement ou devient chargé négativement lorsqu'il est humidifié par un fluide aqueux d'un pH supérieur à 2, et/ou sur une surface, et de préférence sur une surface extérieure du corps, et d'une manière plus préférée sur au moins 50 % de la surface extérieure du corps.

5. Un cathéter intermittent tel que revendiqué dans l'une quelconque des revendications précédentes, dans lequel le polymère du cathéter intermittent comprend au moins un radical, qui est chargé négativement ou qui devient chargé négativement, lorsqu'il est humidifié par un fluide aqueux d'un pH supérieur à 2, dans lequel le au moins un radical est de préférence présent dans le polymère du cathéter intermittent en une concentration totale d'au moins 0,5 % en poids, et dans lequel au moins l'un des radicaux est présent, de préférence à et/ou sur une surface du corps, de préférence sur la surface extérieure, et d'une manière encore plus préférée sur au moins 50 % de la surface extérieure du corps, et dans lequel au moins 50 % des radicaux sont, de préférence, à et/ou sur la surface extérieure du corps.

6. Un cathéter intermittent tel que revendiqué dans l'une quelconque des revendications précédentes, dans lequel le polymère comprend au moins un radical ionisable, de préférence au moins un radical déprotonisable, qui devient chargé négativement, lorsqu'il est humidifié par un fluide aqueux d'un pH supérieur à 2.

7. Un cathéter intermittent tel que revendiqué dans l'une quelconque des revendications précédentes, dans lequel le au moins un additif est un copolymère séquencé A-B comprenant une séquence A hydrocarbonée hydrophobe et une séquence B hydrophile.

8. Un cathéter intermittent tel que revendiqué dans la revendication 7, dans lequel la séquence A comprend une séquence en chaîne hydrocarbonée de formule CH₃CH₂ (CH₂CH₂)ₐ, dans laquelle **"a"** va de 5 à 25, en allant de préférence de 9 à 25.

9. Un cathéter intermittent tel que revendiqué dans la revendication 7 ou 8, dans lequel la séquence B est un oligomère hydrophile comprenant entre 2 et 10 motifs monomères, éventuellement dérivé d'au moins un monomère choisi indépendamment dans le groupe consistant en : des oxydes d'alcoylène, des alcoylène glycols, des épihalohydrines, des acides carboxyliques insaturés, des alcoylène imines, des lactones, de l'alcool vinylique et des vinyl alcanoates.

10. Un cathéter intermittent tel que revendiqué dans l'une quelconque des revendications précédentes, dans lequel le au moins un additif est réparti homogènement avec le polymère.

11. Un cathéter intermittent tel que revendiqué dans l'une quelconque des revendications précédentes, dans lequel le au moins un additif comprend une couche, qui est sur une surface ou comprend une surface du corps, de préférence la surface extérieure.

12. Un cathéter intermittent tel que revendiqué dans l'une quelconque des revendications précédentes, dans lequel le au moins un additif est à et/ou sur au moins 50 % de la surface extérieure du corps tubulaire polymère.

13. Un cathéter intermittent tel que revendiqué dans l'une quelconque des revendications précédentes, dans lequel le polymère, qui est chargé négativement ou qui devient chargé négativement lorsqu'il est humidifié par un fluide aqueux d'un pH supérieur à 2 est le polymère du corps du cathéter, et dans lequel le polymère du corps du cathéter est éventuellement un dérivé d'au moins une matière polyoléfinique.

14. Un procédé de fabrication d'un cathéter intermittent suivant l'une quelconque des revendications 1 à 13, comprenant le stade de : incorporer un radical dans un polymère de cathéter intermittent comprenant au moins une matière polyoléfinique et/ou au moins une matière élastomère thermoplastique, dans lequel le radical est chargé négativement ou devient chargé négativement lorsqu'il est humidifié par un fluide aqueux d'un pH supérieur à 2.

15. Utilisation d'un dérivé de polymère d'au moins une matière polyoléfinique et/ou d'au moins une matière élastomère thermoplastique, qui est chargé négativement ou qui devient chargé négativement lorsqu'il est humidifié par un fluide aqueux d'un pH supérieur à 2, comme répulsif des bactéries et/ou lubrifiant dans ou sur un cathéter intermittent suivant l'une quelconque des revendications 1 à 13.
